(19) **Europäisches Patentamt**

**European Patent Office**

**Office européen des brevets**

(11) Publication number: **0 256 640 B1**

(12) **EUROPEAN PATENT SPECIFICATION**

(45) Date of publication of patent specification: **03.06.92**   (51) Int. Cl.⁵: **A61M 1/28**

(21) Application number: **87305518.0**

(22) Date of filing: **22.06.87**

(54) **Connecting device for peritoneal dialysis.**

(30) Priority: **20.06.86 GB 8615056**

(43) Date of publication of application:
**24.02.88 Bulletin 88/08**

(45) Publication of the grant of the patent:
**03.06.92 Bulletin 92/23**

(84) Designated Contracting States:
**AT BE CH DE ES FR GB GR IT LI NL SE**

(56) References cited:
**EP-A- 0 163 811**
**US-A- 4 306 976**
**US-A- 4 473 369**

(73) Proprietor: **BIEFFE MEDITAL S.A.**
**Via Balestra, 27**
**CH-6900 Lugano(CH)**

(72) Inventor: **Merry Del Val Domingo**
**c/o Medital S.A. Via S.Balestra 27**
**CH-6900 Lugano(CH)**

(74) Representative: **Hedley, Nicholas James Matthew**
**Stephenson Harwood One, St. Paul's Churchyard**
**London EC4M 8SH(GB)**

Rank Xerox (UK) Business Services

## Description

The present invention relates to a device for use in peritoneal dialysis; peritoneal dialysis is a medical technique in which solution for dialysis is conveyed by means of an inlet tube to a peritoneal catheter and from thence to the peritoneal cavity; the solution is left in the peritoneum for the time required for the osmotic dialysis process to take place, and is then drained by way of the same or a different conduit to the outside. This is achieved using a device to connect the catheter to the charging and draining lines. It is usual in a single session to drain the peritoneal cavity and then to fill it again with dialysis fluid.

The present invention provides a system for connecting a peritoneal catheter to one or more bags or sacks for effecting peritoneal dialysis.

Clinical statistics show that many septic complications arise from the opening of different sets of implements (in the different methodologies), and attempts have been made to avoid these drawbacks in current practice by having recourse to devices and/or prostheses which are filled with disinfectant, for example see US 4 306 976 which describes a system for supplying fresh dialysis liquid to, and draining used dialysis liquid from, a peritoneal catheter. In the system of US 4 306 976 the catheter has at its outer end a chamber filled with a sterilising liquid and held by two rupturable membranes that form walls of the chamber. The system includes a charge line connected via respective valves both to a bag containing fresh dialysis liquid and to a bag for holding used dialysis liquid. The charge line is connected to the catheter by means of a hollow spike at the end of the charge line that can penetrate through the two membranes of the chamber so that fluid can flow between the catheter and the charge line through the hollow spike.

The first aim of the invention is that of providing a universal system (which we shall call simply 'Sayfter system') for making the connections in peritoneal dialysis.

Another aim of the invention is to provide a system and a methodology which leaves the patient free from encumbrances in the form of empty bags, lines, prostheses, etc. between one change and the next, and thus during the period in which the dialytic liquid stays or remains in the peritoneal cavity, known as the 'dwell period'.

Another aim of the invention is to provide optimum antiseptic conditions for the purpose of eliminating septic peritonitis; this is achieved by means of the connection of the catheter to the 'inflow-drainage' system through sterile devices.

Another aim is that of preventing the possibility of the peritoneium coming into direct contact, whether by mistake or due to incorrect operation, with the outside atmosphere, thereby avoiding possible contamination.

According to the present invention, there is provided a system for supplying fresh dialysis liquid to a peritoneal catheter line and thence to a patient's peritoneum and for draining used dialysis liquid from the peritoneum to a drainage line, which system comprises means for connecting the end of the charge line and/or the drainage line to the end of the catheter line, said connecting means comprising a spike, which is preferably hollow, provided at one of the said ends (preferably the end of the inflow/drainage line) and a chamber, which preferably contains a sterilizing medium, provided on the other of the said ends (preferably the end of the catheter line), which chamber has a membrane that can be ruptured by the spike to provide fluid communications between the two ends. The system also includes means for opening and closing the catheter line, which means can be in an open state in which it allows fluid to flow down the catheter line or in a closed state in which such fluid flow is prevented, which means is such that it cannot be opened or maintained in the open state manually without the use of a key and wherein the system further includes a key secured to the end of the inflow/drainage line, whereby the end of the catheter line cannot be separated from the end of the inflow/drainage without first removing the key from the opening/closing means and thereby closing the catheter line.

The inflow line may be connected to any container, for example to a bag, containing dialysis liquid. The connection between the inflow line and the rest of the system may be made and sterilised a considerable time before dialysis, e.g. in a factory, or it may be made and sterilised on the spot.

Likewise, the drainage line may be connected to a rigid or soft discharge receptacle for receipt of spent dialysis liquid; the connection between the drainage line and the rest of the system may be made at any time before dialysis.

The system may include a sterile compartment within which the spike is housed, which compartment has a rupturable wall through which the spike can penetrate.

In a preferred embodiment of the system, the fluid communication provided between the ends of the two lines is such that dialysis fluid passes through the chamber that, before rupturing of the said membrane, contained sterilizing medium.

The chamber is preferably such that it can be detached from its respective end and replaced by a fresh container; for this the chamber may be provided with a second membrane and the respective end can be provided with a spike which can rupture the second membrane in the chamber to provide fluid communication between the interior of the chamber and the said respective end.

By means of known flow regulators, the dialysis liquid can be drained from the peritoneum through the catheter line through the chamber and into the drainage line. Filling the peritoneum with fresh fluid takes place similarly (but in reverse).

When charging is finished, the opening/closing means on the catheter line should be closed before the end of the inflow line is detached from the end of the catheter line; this is achieved automatically in the system of the present invention since it is not possible to separate these two ends without first disengaging the key from the opening/closing means and hence closing the catheter line. After disconnecting, the chamber may be discarded or set aside to be recovered and sterilised.

The ends of the catheter line and of the inflow/outflow lines are sterilised by the fluid in the chamber. A notable feature of the system is the fact that all the joining members and elements are sterile at the moment of connection.

Another feature is that the members and elements mentioned above are connected in a sterile and sterilising environment.

Methods for peritoneal dialysis are known which use either a single plastic bag or two bags. The present system can be used equally with one bag or with two bags or with any other type of container, increasing the fields of use and reducing possible peritoneal complications.

The various aspects and advantages of the invention will now be described in further detail, by way of example only, with reference to the accompanying drawings which show in schematic and partial longitudinal section, the various elements of the system.

In Figures I to 6, the references indicate respectively:
LC: charge or inflow line for fresh dialysis liquid
LS: discharge or drainage line for spent dialysis liquid
CL: line to the peritoneal catheter.
Regulators KI and K2 are provided on both lines LC and LS.

In a preferred embodiment, the present invention comprises the following four basic elements:

(a) an element SL, which we will hereinafter call a "Sayfter-lock", and which is connected to the ends of the inflow line LC and the drainage line LS,

(b) an element F, which we will hereinafter call a "Flutlock", and which is connected to the end of the catheter line CL,

(c) an element SC, which we will hereinafter call a "Sayfter-cap", and which is connected to the Flutlock F at the end of the catheter line CL, and

(d) a clamping element K, which we will hereinafter call a "Keyclamp", for clamping the catheter line CL.

The Sayfter-lock SL consists of:
- a base SLB (Sayfter-lock base)
- a connector SLC in the form of a hollow spike (Sayfter-lock connector)
- a protective sheath SLP (Sayfter-lock protector)
- a membrane SLM (Sayfter-lock membrane) and
- an internally-threaded nut SLN (Sayfter-lock nut).

The Sayfter-cap SC has the following parts in the preferred embodiment:
- an externally threaded body SCB (Sayfter-cap body)
- a connecting membrane SCM (Sayfter-cap membrane)
- a connecting membrane SCFM to the Flutlock F (Sayfter-cap/Flutlock membrane)
- a female threaded connector SCN (Sayfter-cap nut)
- sterilizing fluid SCS (Sayfter-cap steriliser).

The Flutlock F is one of the most important parts of the system because it is not exchanged between one dialysis session and the next and is connected directly to the catheter. It should preferably be made of titanium (or of a similar light, stainless material) for reasons of asepsis, accuracy, durability and weight. Its parts are:
- a connector FC Flutlok connector
- a threaded body FB Flutlock body
- an anatomical handgrip FG Flutlock grip
- a connection to the catheter FCA Flutlock catheter adaptor.

The inflow and drainage lines LC and LS are connected to the Sayfter-lock SL by means of suitable seats on the base of a junction piece SLB which also serves as a handgrip so that the Sayfter-lock SL can be easily engaged with the Sayfter-cap SC by means of the female thread SLN on the Sayfter-lock engaging with a male thread on the Sayfter-cap SC. The Sayfter-lock nut SLN turns freely around the connector LC and serves to introduce it gently and without any force into the Sayfter-cap SC.

The Sayfter-lock connector LC is a hollow needle or spike which, passing through the membrane SLM, penetrates the Sayfter-cap membrane SCM thereby allowing the sterilizing fluid to contact the internal Sayfter-lock parts and thereby sterilise them. The sheath SLP and the membrane SLM serve to guarantee the sterility and integrity of the Sayfter-lock.

The Sayfter-cap SC has a cylindrical body SCB but it may be a different shape; for example it may have the shape of a parallelepiped, an ellipsoid or paraboloid; the body may be made of rigid or, more preferably, of soft material, for example it may be a small capsule of soft or semi-rigid plastics material.

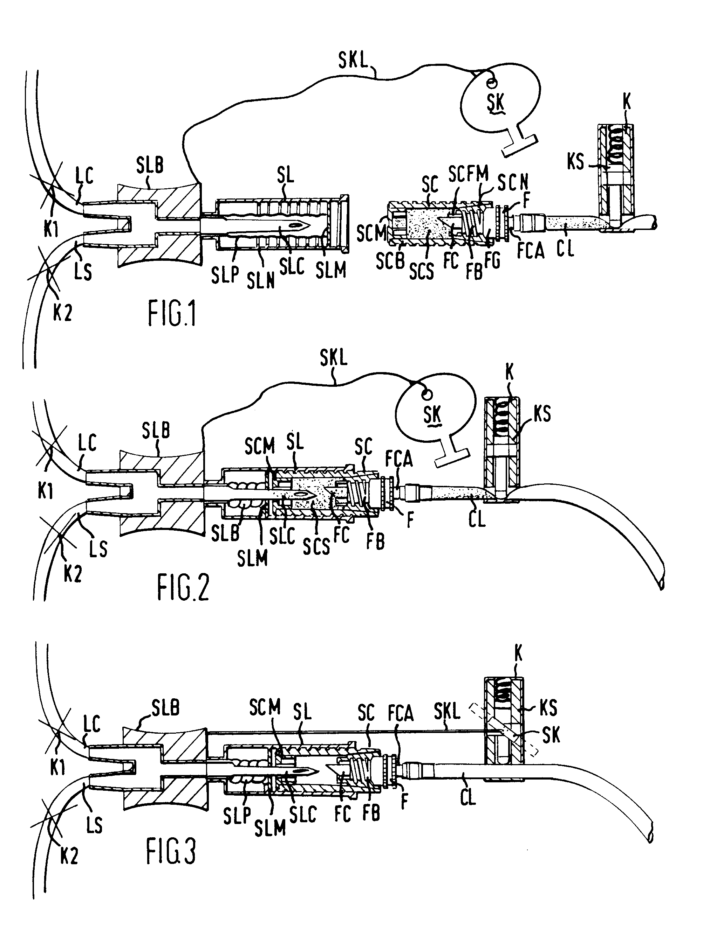

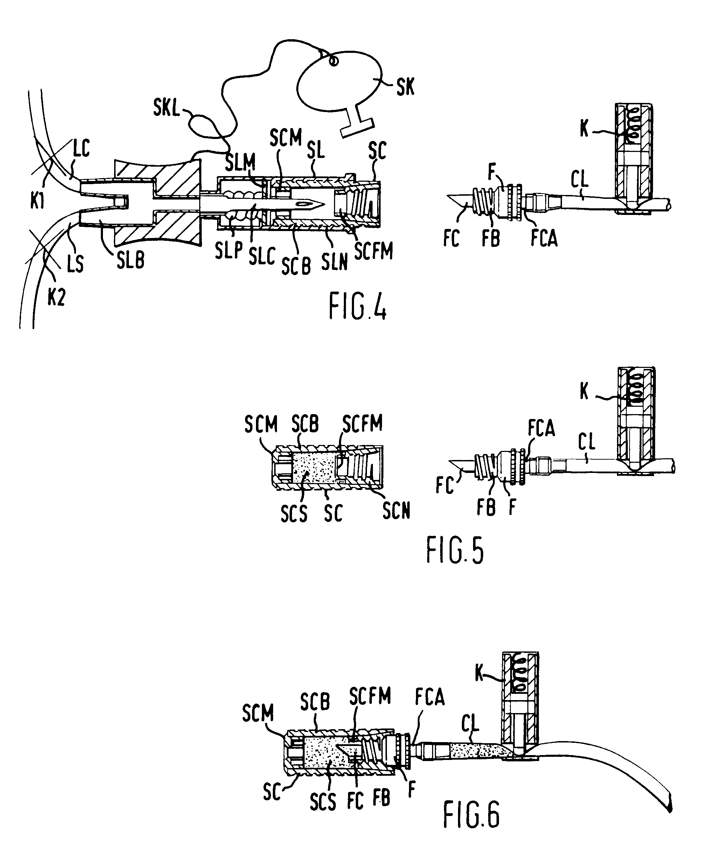

The Sayfter cap SC has the two-fold function of (a) sterilizing the internal flow passages in the Flutlock connector FC and in the Sayfter-lock connector SLC before a change of dialysis fluid takes place (Figure 2) and (b) protecting the Flutlock during the dwell period between one change and the next (Figure 6).

The sterilizing fluid may be, for example, of the povidone iodine type, or sodium hypochlorite, quaternary ammonia, diphenol or other sterilizing fluids already used in other similar cases.

The Keyclamp K includes of a clamping element KS which is biassed by a spring to compress the catheter line and prevent flow therein. The Keyclamp cannot be opened manually but it can be opened with the aid of a key SK which is attached by a line SKL to the base SLB of the Sayfter-lock SL.

Figure I shows the system immediately before a change-over of dialysis liquid and at this stage, the Sayfter-cap SC is connected to the Flutlock F. To effect a change of dialysis liquid, the Sayfter-lock SL is brought close to the Sayfter-cap/Flutlock assembly SC/F and the nut SLN is screwed onto the external thread SCB of the Sayfter-cap thereby pushing the Sayfter-lock connector or spike SLC gently through membrane SCM and into the sterilization chamber of the Sayfter-cap. In this way, the arrangement shown in Figure 2 is reached which is maintained for sufficiently long to effect sterilization.

Before the Sayfter-lock SL is connected to the Sayfter cap SC and during the sterilisation period, as described above in connection with Figs I and 2, the Keyclamp K clamps the line CL between the Flutlock F and the catheter and prevents any flow along the catheter line CL.

As stated above, the Keyclamp K may have one or more movable elements or members KS which interupt the liquid flow in line CL, which is made of elastic plastics material. For example, the Keyclamp K can be composed of a parallelepipedal, ellipsoid or cylindrical body, equipped with a clamping element KS which slides within the body and acts on the catheter line to clamp it. The clamping element is urged by a spring. The Keyclamp can only be released from its clamping position shown in Figures I and 2 by the key SK which is attached to the Sayfter-lock SL by line SKL.

Alternatively, the clamp K may have two hinged jaws which clamp the tube CL between them and which are connected together at their ends opposite the hinge by snap closure elements which can only be opened by a key attached to the Sayfter-lock SL in an analogous manner to that shown in Figures I and 2.

The Keyclamp K thus clamps the catheter line CL at all times when the Sayfterkey SK is removed and detached from the Keyclamp K. Similarly, the opening of the line CL to the catheter, and therefore the drainage of liquid from the peritoneum (not illustrated), is possible only after the insertion of this key SK into the Keyclamp K.

As mentioned above, the Sayfterkey SK is fastened and anchored to the Sayfter-lock SL so that it can only open the Keyclamp K when the Sayfter-lock SL has been firmly connected to the Sayfter-cap/Flutlock assembly. In this way, the possibility of the peritoneum coming into direct contact with the external atmosphere - even due to incorrect operation or some other fortuitous occurrence - is avoided, thereby reducing the risk of contamination. Also, the illustrated arrangement does not allow the Sayfter-lock/Sayfter-cap assembly SL/SC to be detached from the Flutlock F without having previously taken out the Sayfterkey SK, and thereby automatically closing the line CL to the peritoneum catheter.

Returning to the description of the operation of the system, after the sterilization period has been completed (Figure 2), the Sayfterkey SK is used to open the clamp K (Figure 3); also the regulators $K_1$, $K_2$ are set to allow fluid in the peritoneal cavity to drain out through drainage line LS.

When drainage is completed, the regulators $K_1$ and $K_2$, or alternatively pincer or similar clamps, are used to close drainage line LS and open inflow line LC which is attached to a bag of fresh dialysis fluid and the phase of charging the fresh fluid through the charge line LC is initiated.

When this operation is completed, the Sayfterkey SK is take out thereby closing the catheter line CL and also making it possible to detach the Sayfter-lock/Sayfter cap assembly SL/SC (Figure 4).

The connection between the Sayfter-lock SL and the Sayfter-cap SC cannot be undone owing to the appropriate external configuration of the body SCB of the Sayfter-cap SC which makes disconnection impossible. In unscrewing the Sayfter-lock SL, therefore, the Sayfter-cap/Flutlock connection SC-F is undone instead (see Figure 4).

The Sayfter-lok/Sayfter-cap assembly SL/SC is discarded or set aside for subsequent recovery and sterilization if it is so desired. A new Sayfter-cap SC is then screwed onto the Flutlock F, thereby closing it off and simultaneously sterilizing it (Figure 5); the new Sayfter-cap remains in place for security and protection until the next change (Figure 6).

It is advisable once a day to wash the external parts of the Flutlock with water and/or mild detergent to remove any possible trace of inert residues (iodine, etc) which may have become deposited.

The invention provides the following substantial advantages:

a) The near impossibility of contamination during the connection phases;

b) The simplicity of operation, which does not require the use of syringes, pumps, bottles or containers from which to take or transfer disinfectants, masks, gloves, special equipment for making the various connections, all of which make peritoneal dialysis not very practical and stand in the way of its widespread use;

c) Safety in use since it eliminates errors in operation which could seriously affect the health of the patient, either because of contamination arising from direct communication of the peritoneum with the external atmosphere, or because of disinfectant entering the peritoneal cavity;

d) Maximum freedom for the patient; with the majority of systems now in use the patient is required to carry around uncomfortable and cumbersome tubes, empty bags, etc., which constitutes another of the principal grounds for opposition to the spread and acceptance of peritoneal dialysis, especially among the young.

The Sayfter-cap SL as described herein can be miniaturised to the size of the cap of a ballpoint pen, or to half the size of a teabag if produced in non-cylindrical form.

Thus, the system of the present invention gives maximum aseptic safety, great simplicity in use, an operating safety which does not permit errors, minimum encumbrance, the re-use of the part fixed to the catheter, low cost, and the possibility of being used with almost any type of peritoneal dialysis containers at present on the market.

In summary, and without limiting the scope of the invention, the device of the invention preferably consists of five major parts:

l) The Sayfterkey SK, the line SKL between the Sayfter-lock SL and the Sayfterkey SK which prevents connection errors and which permits the opening of the Keyclamp K only when the system has been fully connected up.

2) The Keyclamp K for closing off the catheter, thereby allowing the catheter line to be closed in such a way that it cannot be opened by mistake. The Keyclamp K can only be opened by insertion of the Sayfterkey SK. Within the scope of the present invention, in place of the Keyclamp K, any type of traditional closure or clamp used to close elastic tube (roller clamps, plate clamps, bar-clamps, etc.) can be used but this would detract from the safety of the system as a whole.

3) The Flutlock F for connecting the catheter CL to the rest of the system. The Flutlock F consists of a connector, preferably made of titanium, and provided with a screw thread with at least three turns, a cutting end or spike FC having a shape similar to the mouthpiece of a flute or the point of a quill pen which serves to perforate the closing membrane SCFM of the Sayfter-cap SC.

4) The Sayfter-cap SC is a perfectly watertight single-use cylinder, containing sterilizing liquid between two membranes SCM and SCFM. In screwing the Sayfter-cap SC onto the Flutlock F, the sterlizer contained therein passes to the inside of the Flutlock F, completely protecting the system from septic infections.

5) The Sayfter-lock SL is a two-way single-use or re-usable sterile connector which is completely closed, formed by a perforator or spike SLC covered by a movable protective sheath SLP which can be pulled back over the perforator with a bellows action. The sheath SLP is connected on the one hand to the base SLB of the connector and on the other hand to a plastic membrane SLM. On the outside there is a movable nut SLN which engages the 'Sayfter cap' SC with the Sayfter-cap SC. The Sayfter-lock SL is connected both to the charge line LC which supplies fresh dialysis liquid and to the drainage line LS for removing spent fluid.

## Claims

1. A system for supplying fresh dialysis liquid to a peritoneal catheter line and thence to a patient's peritoneum and for draining used dialysis liquid from the peritoneum to a drainage line, which system comprises means for connecting the end of the charge line to the end of the catheter line (CL), said connecting means comprising a spike (SLC) provided at one of the said ends and a chamber (SC), which preferably contains a sterilizing medium (SCS), provided on the other of the said ends, which chamber has a membrane (SCM) that can be ruptured by the spike (SLC) to provide fluid communications between the two ends characterised in that the system includes means (K) for opening and closing the catheter line (CL), which means can be in an open state in which it allows fluid to flow down the catheter line or in a closed state in which such fluid flow is prevented, which means (K) is such that it cannot be opened or maintained in the open state manually without the use of a key (SK) and wherein the system further includes a key secured to the end of the inflow/drainage line, whereby the end of the catheter line (CL) cannot be separated from the end of the inflow/drainage line (LC, LS) without first removing the key from the opening/closing means and thereby closing the catheter line.

**2.** A system as claimed in claim 1, which includes a sterile compartment (SLP) within which the spike (SLC) is housed, which compartment has a rupturable wall (SLM) through which the spike can penetrate.

**3.** A system as claimed in claim 2, wherein the rupturable wall (SLM) is part of a sheath (SLP) enclosing the spike (SLC), which sheath can be pulled back over the spike.

**4.** A system as claimed in any one of claims 1 to 3, wherein the fluid communication provided between the said two ends is such that dialysis fluid passes through the chamber (SC) that, before rupturing of the said membrane (SCM), contained sterilizing medium (SCS).

**5.** A system as claimed in any one of claims 1 to 4, wherein the catheter line (CL) is made of compliant material and wherein the opening/closing means (K) is a clamp for clamping the catheter line, the clamp comprising a pair of clamping elements and means for holding or biassing the two clamping elements together.

**6.** A system as claimed in any one of claims 1 to 5, wherein the chamber is detachable secured to its respective end, which end includes a spike (FC) and which chamber includes a second membrane (SCFM) that is rupturable by the said spike to provide fluid communication between the interior of the chamber and the said end.

**7.** A system as claimed in any one of claims 1 to 6, wherein the inflow and/or drainage lines (LS, LC) are fitted with a closable valve ($K_1$, $K_2$).

**8.** A system as claimed in any one of claims 1 to 7, wherein a single line serves as the inflow/drainage lines or wherein the system includes separate inflow/drainage lines.

**9.** A system as claimed in any one of claims 1 to 8, wherein the spike (SLC) is provided at the end of the inflow/drainage lines (LC/LS) and the chamber (SC) is provided at the end of the catheter line (CL).

**Revendications**

**1.** Système pour alimenter en liquide de dialyse frais un conduit de cathéter péritonéal, et donc le péritoine d'un patient, et pour drainer le liquide de dialyse utilisé depuis le péritoine vers un conduit de drainage, lequel système comprend un moyen pour connecter l'extrémité du conduit de charge à l'extrémité du conduit de cathéter (CL), ledit moyen de connexion comportant une pointe (SLC) à l'une de ses extrémités, et une chambre (SC) qui contient de préférence un milieu stérilisant (SCS) prévue à l'autre de ses extrémités, laquelle chambre comporte une membrane (SCM) qui peut être rompue par la pointe (SLC) pour réaliser une communication de fluide entre les deux extrémités, caractérisé en ce que le système comprend des moyens (K) pour ouvrir et fermer le conduit de cathéter (CL), lesquels moyens peuvent être en un état ouvert dans lequel ils permettent au fluide de s'écouler le long du conduit de cathéter, ou dans un état fermé dans lequel un tel écoulement de fluide est empêché, lesquels moyens (K) sont tels qu'ils ne peuvent pas être ouverts ou maintenus à l'état ouvert manuellement sans l'utilisation d'une clé (SK) et en ce que le système comprend, en outre, une clé fixée à l'extrémité du conduit alimentation/drainage, grâce à quoi l'extrémité du conduit de cathéter (CL) ne peut pas être séparée de l'extrémité du conduit alimentation/drainage (LC, LS) sans, au préalable, enlever la clé des moyens d'ouverture/fermeture et, par conséquent, fermer le conduit de cathéter.

**2.** Système selon la revendication 1 qui comporte un compartiment stérile (SLP) à l'intérieur duquel est logée la pointe (SLC), lequel compartiment présente une paroi ruptible (SLM) au travers de laquelle la pointe peut pénétrer.

**3.** Système selon la revendication 2 dans lequel la paroi ruptible (SLM) fait partie d'un manchon (SLP) entourant la pointe (SLC), lequel manchon peut être tiré vers l'arrière par dessus la pointe.

**4.** Système selon l'une quelconque des revendications 1 à 3, dans lequel la communication de fluide réalisée entre lesdites deux extrémités est telle que le fluide de dialyse passe au travers de la chambre (SC) qui, avant rupture de ladite membrane (SCM), contient le milieu stérilisant (SCS).

**5.** Système selon l'une quelconque des revendications 1 à 4, dans lequel le conduit de cathéter (CL) est fait d'un matériau déformable et dans lequel les moyens d'ouverture/fermeture (K) sont constitués d'une pince pour pincer le conduit de cathéter, la pince comprenant une paire d'éléments de pinçage et des moyens de maintien ou de contrainte des deux éléments de pinçage l'un vers l'autre.

6.  Système selon l'une quelconque des revendications 1 à 5, dans lequel la chambre est fixée de manière amovible à son extrémité respective, laquelle extrémité comprend une pointe (FC) et laquelle chambre comprend une seconde membrane (SCFM) qui est ruptible par ladite pointe pour réaliser une communication de fluide entre l'intérieur de la chambre et ladite extrémité.

7.  Système selon l'une quelconque des revendications 1 à 6, dans lequel les conduits d'alimentation et/ou de drainage (LS, LC) sont pourvus de valves fermables ($K_1$, $K_2$).

8.  Système selon l'une quelconque des revendications 1 à 7, dans lequel un conduit unique sert de conduits d'alimentation/drainage ou dans lequel le système comprend des conduits d'alimentation/drainage séparés.

9.  Système selon l'une quelconque des revendications 1 à 8, dans lequel la pointe (SLC) est prévue à l'extrémité des conduits d'alimentation/drainage (LC/LS) et la chambre (SC) est prévue à l'extrémité du conduit de cathéter (CL).

**Patentansprüche**

1.  System zum Zuführen frischer Dialyseflüssigkeit in eine peritoneale Katheterleitung und damit zu dem Peritoneum eines Patientens und zum Ableiten gebrauchter Dialyseflüssigkeit vom Peritoneum in eine Drainageleitung, wobei das System Mittel umfaßt, um das Ende der Zuführleitung an das Ende der Katheterleitung (CL) anzuschließen, wobei diese Verbindungsmittel einen Dorn (SLC) umfassen, der an einem der besagten Enden vorgesehen ist und eine Kammer (SC), die bevorzugterweise eine sterilisierende Flüssigkeit (SCS) enthält und die an dem anderen der besagten Enden vorgesehen ist, wobei die Kammer eine Membran (SCM) aufweist, die durch den Dorn (SLC) durchstoßbar ist, um eine Strömungsverbindung zwischen den beiden Enden zu ermöglichen,
dadurch gekennzeichnet,
daß das System eine Vorrichtung (K) zum Öffnen und Schließen der Katheterleitung (CL) enthält, wobei die Vorrichtung in einer Offenstellung sein kann, die es erlaubt, daß Flüssigkeit die Katheterleitung entlangfließen kann, oder die in einer Geschlossenstellung sein kann, bei der ein solches Fließen von Flüssigkeit verhindert wird, wobei die Vorrichtung (K) so ist, daß sie ohne den Gebrauch eines Schlüssels (SK) manuell nicht geöffnet oder in der Offenstellung gehalten werden kann und wobei das System außerdem einen Schlüssel umfaßt, der an dem Ende der Zuführ-/Ableitleitung festgemacht ist, wobei das Ende der Katheterleitung (CL) nicht vom Ende der Zuführ-/Ableitleitung (LC, LS) getrennt werden kann ohne erst den Schlüssel aus der Öffnungs-/Schließvorrichtung zu entfernen und dadurch die Katheterleitung zu schließen.

2.  System gemäß Anspruch 1
dadurch gekennzeichnet,
daß es eine sterile Kammer (SLP) aufweist, in der der Dorn (SLC) angeordnet ist, wobei die Kammer eine durchstoßbare Wand (SLM) hat, durch die der Dorn hindurchtreten kann.

3.  System gemäß Anspruch 2,
dadurch gekennzeichnet,
daß die durchstoßbare Wand (SLM) Teil einer Hülle (SLP) ist, die den Dorn (SLC ) umschließt, wobei diese Hülle über den Dorn zurückgezogen werden kann.

4.  System gemäß einem der Ansprüche 1 bis 3,
dadurch gekennzeichnet,
daß die Strömungsverbindung, die zwischen den beiden besagten Enden vorgesehen ist, derart ist, daß die Dialyseflüssigkeit die Kammer (SC) passiert, die vor Zerbrechen der besagten Membran (SCM) sterilisierende Flüssigkeit (SCS) enthielt.

5.  System gemäß einem der Ansprüche 1 bis 4,
dadurch gekennzeichnet,
daß die Katheterleitung (CL) von entsprechendem Material ist und daß die Öffnungs-/Schließvorrichtung (K) eine Klammer ist, um die Katheterleitung abzuklemmen, wobei die Klammer ein Paar von Klemmelementen umfaßt und Mittel um die beiden Klemmelemente zusammenzuhalten oder zusammenzuspannen.

6.  System gemäß einem der Ansprüche 1 bis 5,
dadurch gekennzeichnet,
daß die Kammer lösbar an ihrem entsprechenden Ende befestigt ist, wobei dieses Ende einen Stachel (FC) aufweist und die Kammer eine zweite Membran (SCFM) hat, die durch den besagten Stachel durchstoßbar ist, um eine Strömungsverbindung zwischen dem Inneren der Kammer und dem besagten Ende zu ermöglichen.

7. System gemäß einem der Ansprüche 1 bis 6,
dadurch gekennzeichnet,
daß die Zuführ- und/oder Ableitleitungen (LS, LC) mit einem verschließbarem Ventil (K₁, K₂) ausgestattet sind.

8. System gemäß einem der Ansprüche 1 bis 7,
dadurch gekennzeichnet,
daß eine einzelne Leitung als Zuführ-/Ableitleitung dient oder daß das System separate Zuführ-/Ableitleitungen umfaßt.

9. System gemäß einem der Ansprüche 1 bis 8,
dadurch gekennzeichnet,
daß der Dorn (SLC) sich am Ende der Zuführ-/Ableitleitung (LC/LS) befindet und sich die Kammer (SC) an dem Ende der Katheterleitungen (CL) befindet.

FIG.1

FIG.2

FIG3

FIG.4

FIG.5

FIG.6